# EUROPEAN PATENT SPECIFICATION

(11) **EP 1 226 834 B1**
(45) Date of publication and mention of the grant of the patent: **03.01.2007**
(21) Application number: 00964723.1
(22) Date of filing: 06.10.2000
(51) Int. Cl.: A61L 2/24, A61L 2/20, A61L 9/015

(54) **FORMALDEHYDE GAS STERILIZER**
FORMALDEHYDGAS-STERILISATOR
STERILISATEUR AU FORMALDEHYDE GAZEUX

(30) Priority: 08.10.1999 JP 28842999; 08.06.2000 JP 2000171795
(43) Date of publication of application: 31.07.2002
(73) Proprietor: Bio Media Co., Ltd., Chiyoda-ku Tokyo 101-0021 (JP); Hashiba, Tomohiko, Tokyo 101-0021 (JP)
(72) Inventor: HASHIBA, Tomohiko, c/o Bio Media Co., Ltd., Tokyo 105-0013 (JP)
(74) Representative: HOFFMANN EITLE
(86) International application number: PCT/JP2000/006990
(87) International publication number: WO 2001/026697

(56) References cited:
- WO-A-97/47331
- FR-A- 2 354 779
- FR-A- 2 502 498
- JP-A- 11 226 094
- US-A- 4 119 400
- US-A- 4 764 351
- US-A- 5 882 590
- DATABASE WPI Section Ch, Week 199944 Derwent Publications Ltd., London, GB; Class D22, AN 1999-374211 XP002221548 & JP 11 226094 A (FEATHER GLASS KK), 24 August 1999 (1999-08-24)

## Description

### TECHNICAL FIELD

The present invention relates to a formaldehyde gas disinfection apparatus whereby a disinfection space is disinfected using formaldehyde gas.

### BACKGROUND ART

Methods of using formaldehyde gas for the purpose of disinfection treatment of spaces in bioclean rooms, operating rooms and the like have been conventionally known whereby a disinfection space is sealed and a formaldehyde gas generator is set therein and used to generate formaldehyde gas.

However, since the disinfecting (throughout the present specification, this term will include the meaning of "sterilizing") effect of formaldehyde gas depends largely on the formaldehyde gas concentration, humidity and temperature in the disinfection space, simply filling the disinfection space with formaldehyde gas for the specified time has not been sufficient to adequately guarantee a disinfecting effect.

In addition, formaldehyde gas must produce a guaranteed disinfecting (and sterilizing) effect not only in spaces to be disinfected which are established in rooms, but also in mobile spaces, temporarily formed spaces and spaces with shapes that do not permit the use of ordinary formaldehyde gas generators. Specifically, such spaces exist in ambulances, temporary tent operating rooms and artificial respirators. It has been difficult to achieve a sufficient disinfecting effect in such spaces by conventional methods. Moreover, such disinfection spaces also require interior pressure control since they are sealed spaces (rooms).

FR-A-2 354 779 discloses a disinfection device which permits disinfecting a closed space by means of formaldehyde gas, wherein the chamber may be brought to an optimal temperature for preventing the formaldehyde from polymerizing.

US-A-5 882 590 describes a system and method for real-time monitoring and control of chemical sterilant concentration during all phases of a sterilization cycle.

JP-A-11 226094 discloses a method for sterilizing closed spaces such as surgery rooms, medical rooms, etc. by means of formaldehyde gas, employing specific ranges of temperature and humidity in the closed space and a certain minimum concentration of the formaldehyde gas.

### DISCLOSURE OF THE INVENTION

One part of the formaldehyde gas disinfection apparatus of the invention may comprise a housing provided with a formaldehyde gas generator which generates formaldehyde gas, a humidity regulator which regulates the humidity of the formaldehyde gas, a temperature regulator which regulates the temperature of the formaldehyde gas, a gas deliverer which delivers and introduces the formaldehyde gas into a disinfection space, a discharge gas treater which treats discharge gas from the disinfection space and a gas ejector which ejects the discharge gas, and provided with a controller which controls generation of the formaldehyde gas in the formaldehyde gas generator at a concentration within a specified range, controls the humidity of the formaldehyde gas within a specified range by the humidity regulator, controls the temperature of the formaldehyde gas within a specified range by the temperature regulator, controls the gas delivery rate by the gas deliverer within a specified range, controls the amount of formaldehyde in the discharge gas by the discharge gas treater within a specified range and controls the amount of discharge gas ejected by the gas ejector, and which controls the formaldehyde gas concentration, humidity and temperature in the disinfection space to 160 ppm or greater, 50-90% RH and 20-40°C, respectively, based on the values of the formaldehyde gas concentration, humidity and temperature in the disinfection space from formaldehyde gas concentration, humidity and temperature monitors in the disinfection space. Incidentally, the formaldehyde gas concentration is the concentration of formaldehyde itself as measured according to JIS K0303 in combination with liquid chromatography analysis.

The formaldehyde gas disinfection apparatus allows delivery to be accomplished in an easy manner while discharge gas is cleaned. Also, since the controller allows the formaldehyde gas concentration, humidity and temperature in the disinfection space to be controlled to 160 ppm or greater, 50-90% RH and 20-40°C, respectively, it is possible to achieve a sufficiently guaranteed disinfecting effect.

The formaldehyde gas disinfection apparatus of the invention comprises a formaldehyde gas generator which generates formaldehyde gas, a humidity regulator which regulates the humidity of the formaldehyde gas, a temperature regulator which regulates the temperature of the formaldehyde gas, a gas deliverer which delivers and introduces the formaldehyde gas into a disinfection space, a discharge gas treater which treats discharge gas from the disinfection space and a gas ejector which ejects the discharge gas, and a controller which controls generation of the formaldehyde gas in the formaldehyde gas generator at a concentration within a specified range, controls the humidity of the formaldehyde gas within a specified range by the humidity regulator, controls the temperature of the formaldehyde gas within a specified range by the temperature regulator, controls the gas delivery rate by the gas deliverer within a specified range, controls the amount of formaldehyde in the discharge gas by the discharge gas treater within a specified range and controls the amount of discharge gas ejected by the gas ejector.

The formaldehyde gas disinfection apparatus according to the invention allows the formaldehyde gas concentration, humidity and temperature in the disinfection space to be controlled to a prescribed concentration, humidity and temperature, respectively, by the controller in order to produce a sufficiently guaranteed disinfecting effect.

The formaldehyde gas disinfection apparatus of the invention is characterized by being provided with a formaldehyde gas supply/ejection apparatus which supplies and ejects formaldehyde gas into and from a sealed chamber and a chamber pressure regulator which regulates the pressure in the chamber, wherein the formaldehyde gas supply/ejection apparatus comprises a formaldehyde gas generator which generates the formaldehyde gas, a humidity regulator which regulates the humidity of the formaldehyde gas, a temperature regulator which regulates the temperature of the formaldehyde gas, a gas deliverer which delivers and introduces the formaldehyde gas into a chamber, a discharge gas treater which treats discharge gas from the chamber, a gas ejector which ejects the discharge gas, and a controller which controls the formaldehyde gas concentration, humidity and temperature in the chamber to a prescribed concentration, humidity and temperature, and the chamber pressure regulator comprises an air supply unit which supplies outside air into the chamber, a gas discharge unit which discharges gas in the chamber to the outside, pressure difference detector means which detects the pressure difference between the inside and outside of the chamber, control means which controls the air supply unit and the gas discharge unit based on a detected value detected by the pressure difference detector means, and optionally controlled state output means which outputs the controlled state of the chamber pressure based on the detected value detected by the pressure difference detector means.

In one embodiment the formaldehyde gas disinfection apparatus of the invention is characterized in that the controller controls the formaldehyde gas concentration in the chamber to 160 ppm or greater based on the outputted value from a formaldehyde gas concentration monitor provided in the chamber, controls the humidity in the chamber to 50-90% (relative humidity) based on the outputted value from a humidity monitor provided in the chamber, and controls the temperature in the chamber to 20-40°C based on the outputted value from a temperature monitor provided in the chamber. Here, the formaldehyde gas concentration is the concentration of formaldehyde itself as measured according to JIS K0303 in combination with liquid chromatography analysis.

The formaldehyde gas disinfection apparatus of the invention is provided with a chamber pressure regulator, thus allowing the chamber pressure to be kept constant even in cases where temperature increase in the chamber causes expansion of the gas in the chamber.

In one embodiment, the formaldehyde gas disinfection apparatus of the invention is characterized in that the gas discharge unit is provided with a treatment apparatus which treats gas discharged from the chamber.

According to this mode of the formaldehyde gas disinfection of the invention, the formaldehyde and other gas in the chamber is treated by a treatment apparatus even in cases where the gas in the chamber has been discharged to regulate the chamber pressure, so that the formaldehyde gas may be treated before it is ejected outside.

### BRIEF DESCRIPTION OF THE DRAWINGS

Fig. 1 is a schematic drawing of a formaldehyde gas supply/ejection apparatus forming part of the disinfection apparatus of the present invention.
Fig. 2 is a schematic drawing of a formaldehyde gas disinfection apparatus according to the present invention.
Fig. 3 is a flow chart illustrating chamber pressure regulation in a formaldehyde gas disinfection apparatus according to the present invention.

### BEST MODE FOR CARRYING OUT THE INVENTION

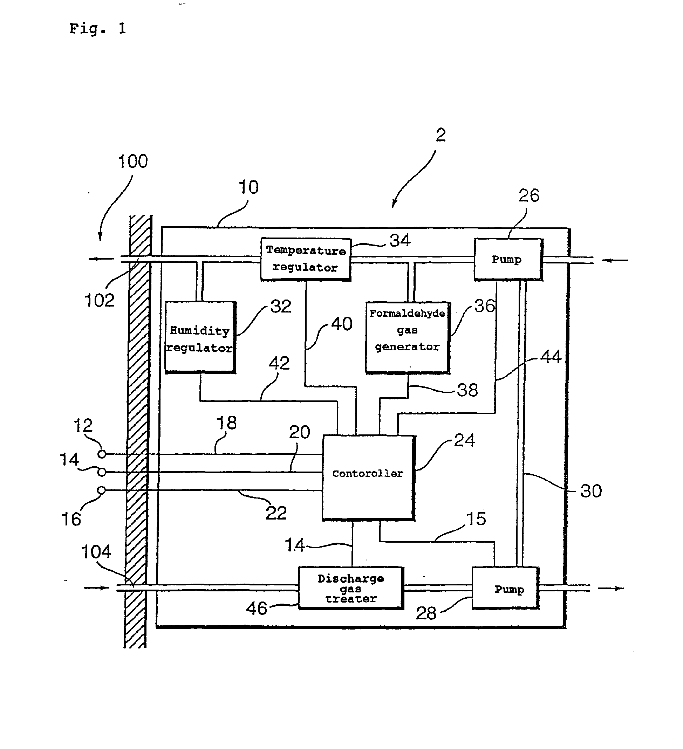

A formaldehyde gas supply/ejection apparatus will now be explained with reference to Fig. 1.

The formaldehyde gas disinfection apparatus 2 has a housing 10 which can be mounted on the outside of a biohazard safety cabinet to allow easy disinfection of the space in the cabinet (hereunder referred to as the "disinfection space 100"). Here, the interior of the cabinet is formed into a closed space by closing a damper or the like. The cabinet is provided with a formaldehyde gas inlet 102 for supply of formaldehyde gas from the formaldehyde gas disinfection apparatus 2, and a discharge gas outlet 104 for ejection of the formaldehyde gas.

In the disinfection space 100 there are provided a formaldehyde gas concentration sensor 12, a humidity sensor 14 and a temperature sensor 16, and the monitored value from each is transmitted to the controller 24 via control lines 18, 20, 22.

Outside air is introduced into the disinfection space 100 through the formaldehyde gas inlet 102 by a pump 26, and then discharged to the outside from the discharge gas outlet 104 through a pump 28. Also, the discharge gas leaving the pump 28 is reintroduced to the pump 26 through a recirculation conduit 30 for circulation of the air in the disinfection space 100.

The temperature and humidity in the disinfection space 100 obtained by the humidity sensor 14 and temperature sensor 16 are regulated by a humidity regulator 32 and temperature regulator 34 of the controller 24 to a specified temperature range of 20-40°C and humidity range of 50-90% (relative humidity). In addition, a prescribed formaldehyde gas concentration of 160 ppm or greater is maintained by regulation with the formaldehyde gas generator 36 and pump 26, and the condition with a formaldehyde gas concentration of 160 ppm or greater is maintained for a prescribed period (5 hours). The formaldehyde gas concentration, humidity and temperature in the disinfection space 100 are each monitored by a concentration sensor 12, humidity sensor 14 and temperature sensor 16, and the necessary calculations are performed by the controller 24 based on these values and the formaldehyde gas generator 36, temperature regulator 34, humidity regulator 32 and pump 26 are controlled through the control lines 38, 40, 42 and 44. The formaldehyde gas concentration is the concentration of formaldehyde itself as measured according to JIS K0303 in combination with liquid chromatography analysis.

After the prescribed time has elapsed, the formaldehyde gas generator 36 is switched off and treatment by the discharge gas treater 46 is carried out until the formaldehyde concentration in the disinfection space 100 falls below a prescribed value. That is, the gas leaving the pump 28 is reintroduced to the pump 26 via the circulation conduit 30 for circulation of the air in the disinfection space 100, thereby gradually reducing the formaldehyde concentration to below the prescribed concentration.

In the mode described above, a state with a formaldehyde gas concentration of 160 ppm or greater is maintained for 5 hours for disinfection of the disinfection space 100, as this condition is the most practical condition. However, the disinfection space 100 may also be disinfected by maintaining a state with a formaldehyde gas concentration of 300 ppm or greater for 2 hours, or by maintaining a state with a formaldehyde gas concentration of 60 ppm or greater for 12 hours.

According to the invention there are no particular restrictions on the formaldehyde gas generator used, but it must be able to generate formaldehyde gas at high concentration with humidity and temperature control.

Since the disinfecting effect of formaldehyde gas is known to be correlated with rise in humidity, the formaldehyde is preferably generated while maintaining optimum humidity. Specifically there may be mentioned means of generating formaldehyde from methanol, including (1) generating means using a catalyst, (2) generating means by ultrasonic treatment and (3) generating means by ultraviolet irradiation. According to the invention, (1) is particularly preferred. Here, a very trace amount of water will be produced as a by-product.

As specific catalysts to be used for the aforementioned generating means using a catalyst, there may be mentioned platinum, copper, aluminum, carbon and mixtures thereof. The catalyst is packed into a cylindrical vessel and the temperature of the cylindrical vessel is regulated for heating and cooling of the catalyst to the prescribed temperature. A prescribed amount of methanol is first gasified and fed to the catalyst section to initiate the catalytic reaction.

Control of the amount of formaldehyde generation depends on control of the catalyst temperature and on the amount of methanol supplied and gasified. Optimization of the reaction conditions is possible by actually generating formaldehyde and appropriately measuring the formaldehyde concentration. Specifically, a calibration curve may be drawn from measurement data for the catalytic reaction temperature and amount of formaldehyde gas generation, with respect to a given methanol supply amount. For example, when 1000 g of methanol is used with copper as the catalyst, 750 g of formaldehyde gas can be produced within about 30 minutes. A gasification method by ultrasonic treatment instead of heating and cooling alone may also be employed for gasification of the methanol.

The formaldehyde generating means is means for ultrasonic treatment or ultraviolet irradiation treatment of methanol. This is made possible by placing the methanol in an appropriate reactor and providing an ultrasonic treatment apparatus or ultraviolet irradiation treatment apparatus around or inside the reactor. According to the invention, the method of generating the formaldehyde gas is preferably a method of heating paraformaldehyde. There are no particular restrictions on the heating means therefor. Also, the amount of gas generated may be easily controlled by controlling the heating time and heating temperature. Paraformaldehyde is ordinarily available as a commercial product. The generated formaldehyde gas may be used directly or after external dilution with a carrier gas (air or inert gas).

The concentration of the formaldehyde gas which is generated by the method of the invention may be easily kept at 160 ppm or greater by using the generator described above. It may also be generated at an even higher concentration to give the disinfecting effect (or sterilizing effect) explained hereunder.

According to the invention, an appropriate formaldehyde gas concentration is preferably held in an appropriate temperature range for a long period in order to regulate the temperature in the disinfection space. There are no particular restrictions on the temperature regulating means provided for this purpose, and any ordinary publicly known heating or cooling apparatus may be employed. Using an apparatus with sufficient heat-exchange volume will allow regulation to a degree permitting variations in temperature to be substantially ignored, although this will depend on the volume and shape of the space. The preferred temperature range for the method of the invention is 20-40°C, and more preferably 25-35°C, as ordinary temperature. However, disinfection can also be accomplished in the disinfection space at temperatures other than these temperature ranges.

According to the invention, an appropriate formaldehyde gas concentration is preferably held in an appropriate humidity range for a long period in order to regulate the humidity in the closed space. There are no particular restrictions on the humidity regulating means provided for this purpose, and any ordinary publicly known humidifying or dehumidifying apparatus may be employed. Using an apparatus with sufficient humidifying or dehumidifying volume will allow regulation to a degree permitting variations in humidity to be substantially ignored, although this will depend on the volume and shape of the space.

The disinfecting effect of formaldehyde gas is known to be dependent on humidity, but when the relative humidity increases above a certain point, condensation occurs due to moisture condensation, and aggregation thereof in the disinfection space causes adhesion of formaldehyde and its oxides such as formic acid and the like on the walls and elsewhere. This results in contamination of the disinfection space.

According to the invention, therefore, the humidity must be maintained at a level in which the disinfecting effect of the formaldehyde is adequately exhibited while the aforementioned condensation phenomenon does not occur. This humidity range will depend on the temperature, but the range is a relative humidity of 50-90% (and preferably 80-90%) in the temperature range of 20-40°C. A humidity below this range results in an insufficient disinfecting effect, and a humidity above this range (above 90%) can result in contamination due to condensation.

There are no particular restrictions on the method of monitoring the temperature in the closed space, and an ordinary thermometer may be used. The monitored temperature may be inputted into the controller either manually or automatically. The temperature in the disinfection space is thus stored for a specific time in the controller.

The precision of the monitored temperature is also not particularly restricted, and it may be a measuring precision of about ±1°C. A plurality of monitoring means may also be employed. This will allow monitoring of variations in the temperature in the chamber, for more precise temperature regulation.

There are no particular restrictions on the method of monitoring the humidity in the closed space, and an ordinary hygrometer may be used. The monitored humidity may be inputted into the controller either manually or automatically. The humidity in the disinfection space is thus stored for a specific time in the controller.

The precision of the monitored humidity is also not particularly restricted, and it may be a measuring precision of about ±1% in a temperature range of 20-40°C. A plurality of monitoring means may also be employed. This will allow monitoring of variations in the humidity in the chamber, for more precise humidity regulation.

There are no particular restrictions on the method of monitoring the formaldehyde concentration in the closed space, and ordinary analysis means may be used. Specifically there may be mentioned a method employing a formaldehyde sensor, or a method by gas chromatography or ion chromatography based on air sampling. The monitored concentration may be inputted into the controller either manually or automatically. The concentration in the disinfection space is thus stored for a specific time in the controller.

The precision of the monitored concentration is also not particularly restricted, and it may be a measuring precision of about ±10 ppm in the aforementioned concentration range. A plurality of monitoring means may also be employed. This will allow monitoring of variations in the concentration in the chamber, for more precise regulation of the concentration.

According to the invention, the temperature, humidity and formaldehyde concentration in the disinfection space must be kept within the prescribed ranges for a prescribed time. The formaldehyde gas concentration in the disinfection space is reduced by various reactions such as the disinfection reaction occurring in the disinfection space. In order to maintain a constant formaldehyde gas concentration, therefore, it is necessary to incorporate the temperature, humidity and formaldehyde concentration data for a set time period and control the formaldehyde generating means for the specific range. There are no particular restrictions on the controlling method or controller used for this purpose, and any manual method or computer program-employing controller may be used. Since a high formaldehyde concentration must be maintained for a long period according to the invention, the controller preferably has a function by which control is achieved by sending signals to the formaldehyde generating apparatus, pump, temperature regulator and humidity regulator while optimizing these signals on an on-time basis.

There are no particular restrictions on the construction of the controller, but it preferably comprises (1) means (such as a keyboard) for inputting the set temperature, set humidity, set formaldehyde concentration, etc., (2) means (such as a memory) for storing the measured data from the temperature, humidity and formaldehyde concentration monitors, (3) means (such as a display or printer) for outputting those values, (4) means for discerning the difference between measured data and the set values and (5) means for outputting control signals to the temperature regulating system, humidity regulating system and formaldehyde gas generating system. For example, when the formaldehyde concentration is determined to be below the set value by the means of (4) above, the means of (5) above sends a control signal to the formaldehyde generating apparatus for supply of the methanol starting material or increase in the catalytic reaction temperature to increase the amount of formaldehyde gas generated.

There are no particular restrictions on the method of measuring the disinfecting effect by the apparatus of the invention, or on the microbes for which it is employed, and any of various publicly known methods may be applied. Specifically there may be mentioned methods conforming to the ISO standard. Commercially available biological indicators in various forms may be used for the invention with high convenience and reproducibility. Specifically there may be used a test strip type (test paper type) or a Proof system, while microbes preferred for application of the invention include Bacillus subtilis, var. niger (ATCC No.9372) and Bacillus stearothermophilus (ATCC No.7953).

The disinfecting effect will usually be judged by (1) placing test strips or the like in a plurality of selected locations in the disinfection space, (2) using the method of the invention for formaldehyde gas disinfection treatment and then (3) culturing the test strips in suitable medium and determining, based on the presence or absence of viable microbes, either negativity (no viable microbes) or positivity (viable microbes). Such a judgment method can thus indicate a disinfecting effect, and even indicate a sterilizing effect.

The culturing conditions used may be, specifically, culturing for 7 days in tryptone-soya bouillon medium (30-35°C ±1.0°C) in the case of bacteria of Bacillus subtilis, var. niger (ATCC No.9372), and culturing for 7 days or longer in tryptone-soya bouillon medium (55-60°C ±1.0°C) in the case of bacteria of Bacillus stearothermophilus (ATCC No.7953).

Specifically, the disinfecting effect obtained by the method of the invention, as determined by the judgment method described above, exhibits negativity (i.e., sterility) when using on the order of 10⁶ cells of Bacillus subtilis, var. niger (ATCC No.9372) and when using on the order of 10⁶ cells of Bacillus stearothermophilus (ATCC No.7953).

There are also no particular restrictions on the treatment or treater for the formaldehyde gas-containing discharge gas which may be used for the invention. There may be mentioned ordinary publicly known treatment methods, such as washing with a scrubber, absorption with an absorbing agent, removal by decomposition reaction using an appropriate catalyst, or combinations of these methods. The degree of treatment and volume of treatment may be easily selected as appropriate in consideration of the concentration of the formaldehyde gas to be treated, the included impurities, the disinfection space volume and emission standards.

The housing of the formaldehyde gas disinfection apparatus of the invention is not particularly restricted, and it facilitates handling as a single unit for installation, removal and transport of the apparatus. It may also be provided with moving means.

There are no particular restrictions on application of the formaldehyde gas disinfection apparatus of the invention, and it may be used for easy and safe disinfection of disinfection spaces such as chamber interiors, chamber exteriors, stationary rooms, mobile rooms and the like. For example, in cases where the disinfection space is a fixed space in a chamber (such as for bioclean rooms, sample preparation clean rooms and operating rooms), the chamber may be sealed and provided with a formaldehyde gas inlet from the chamber and discharge gas outlet to the outside. When the disinfection space is a temporarily defined space outdoors (for example, in an ambulance, mobile clean room, mobile operating room or tent operating room), the formaldehyde gas disinfection apparatus of the invention may be sealed in the same manner and provided with a formaldehyde gas inlet from the chamber and discharge gas outlet to the outside. When the disinfection space is very narrow and long (for example, an artificial respirator), the formaldehyde gas disinfection apparatus of the invention may be provided with a formaldehyde gas inlet at one end for introduction of the formaldehyde gas, thus allowing disinfection of the interior space.

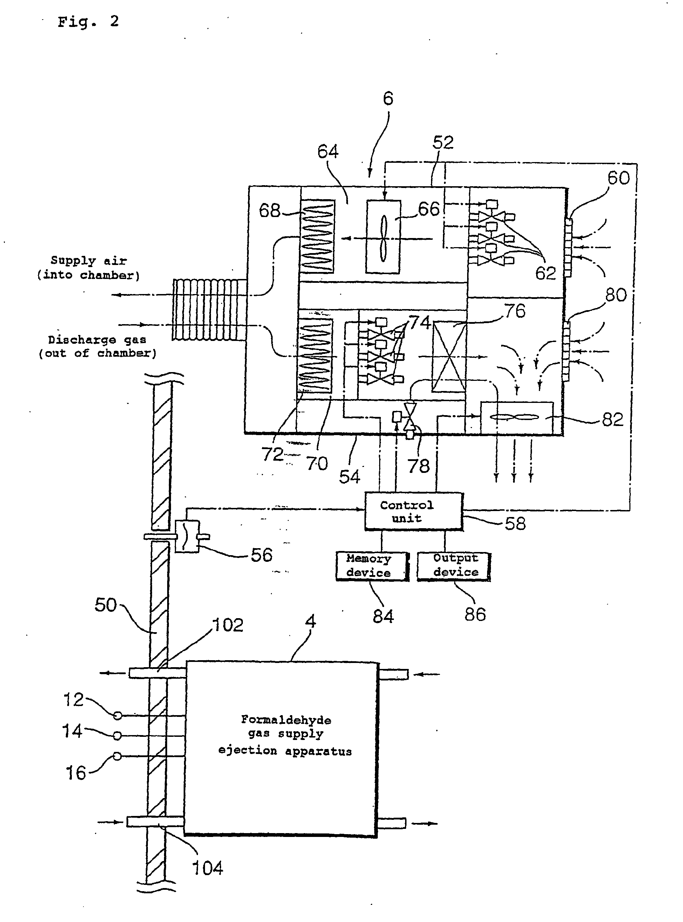

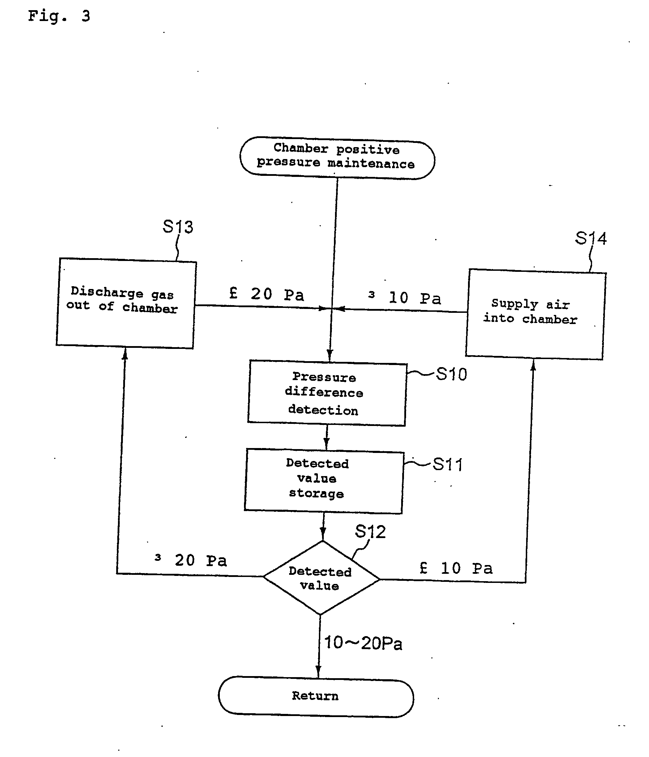

The formaldehyde gas disinfection apparatus according to the invention will now be explained with reference to Figs. 2 and 3. The formaldehyde gas disinfection apparatus according to the invention is provided with a formaldehyde gas supply/ejection apparatus 4 having the same construction as the formaldehyde gas supply/ejection apparatus of Fig. 1, and a chamber pressure regulator 6 which regulates the pressure in the disinfection space formed as a sealed space.

Fig. 2 is a schematic diagram of a formaldehyde gas disinfection apparatus according to the invention, having a construction provided with formaldehyde gas supply/ejection apparatus 4 and a chamber pressure regulator 6. The chamber pressure regulator 6 is provided in contact with the chamber wall 50, and it regulates the pressure in the chamber sealed by the chamber wall 50. The chamber pressure regulator 6 has a construction provided with an air supply unit 52 which supplies air from the outside into the chamber, a gas discharge unit 54 which discharges gas in the chamber to the outside, a fine pressure difference detector 56 which detects pressure changes between the inside and outside of the chamber, and a control unit 58 which controls the air supply unit 52 and gas discharge unit 54 based on the values detected by the fine pressure difference detector 56.

The air supply unit 52 has a supply air grill 60 for incorporation of outside air, and is provided with three supply air regulating electromagnetic valves 62 downstream from the supply air grill 60 for regulation of the air volume supplied from the outside into the chamber. An air fan 66 and HEPA (high efficiency particulate air) filter 68 are provided in that order in an air duct 64 downstream from the supply air regulating electromagnetic valves 62.

The gas discharge unit 54 has a HEPA filter 72 in an air duct 70, and is provided with three discharge gas volume regulating electromagnetic valves 74 downstream from the HEPA filter 72 for regulation of the air volume discharged from the inside to the outside of the chamber. An air treater 76 having a construction provided with a platinum catalyst and a heater is also provided downstream from the discharge gas volume regulating electromagnetic valves 74. Here, outside air is supplied to the air treater 76 through an electromagnetic valve 78. The temperature of the catalyst may be kept constant by supply of this outside air.

Downstream from the discharge gas volume regulating electromagnetic valves 74 there is provided an air fan 82 which discharges air passing through the air treater 76 and air taken in from the supply air grill 80 out from the chamber pressure regulator 6.

The fine pressure difference detector 56 is provided on the chamber wall 50 and connected to the control unit 58 via a signal wire, and the pressure difference between the outside and inside of the chamber detected by the fine pressure difference detector 56 is inputted to the control unit 58.

The control unit 58 is connected to supply air volume regulating electromagnetic valves 62 and the air fan 66 of the air supply unit 52 through a signal wire, and is also connected to the discharge gas volume regulating electromagnetic valves 74 electromagnetic valve 78 and air fan 82 of the gas discharge unit 54. The control unit 58 controls the supply air volume regulating electromagnetic valves 62, air fan 66, discharge volume regulating electromagnetic valves 74 and air fan 82, based on the detected value of the fine pressure difference detector 56. There are also connected to the control unit 58 a storage device 84 which constantly stores the detected value from the fine pressure difference detector 56 and an output device 86 such as a printer which outputs the detected value stored in the storage device 84.

In this formaldehyde gas disinfection apparatus, outside air is introduced into the chamber through a formaldehyde gas inlet 102 by the pump 26 of the formaldehyde gas supply/ejection apparatus 4, and is discharged to the outside air by a pump 28 through a discharge gas outlet 104. The temperature and humidity in the chamber obtained by the humidity sensor 14 and temperature sensor 16 are controlled by the controller 24 to ranges of a temperature of 20-40°C and a humidity of 50-90% (relative humidity), respectively, and are regulated by the humidity regulator 32 and temperature regulator 34. Also, the prescribed formaldehyde gas concentration of 160 ppm or greater is regulated by the formaldehyde gas generator 36 and pump 26 and maintained for the prescribed time period. The formaldehyde gas concentration, humidity and temperature are each monitored by the concentration sensor 12, humidity sensor 14 and temperature sensor 16, the necessary calculations are performed by the controller 24 based on the obtained values, and the formaldehyde gas generator 36, temperature regulator 34, humidity regulator 32 and pump 26 are controlled via the control lines 38, 40, 42, 44. The formaldehyde gas concentration is the concentration of formaldehyde itself as measured according to JIS K0303 in combination with liquid chromatography analysis.

The pressure in the chamber is kept at positive pressure by the chamber pressure regulator while the temperature, humidity and formaldehyde gas concentration in the chamber are kept in a range of 20-40°C, a range of 50-90% (relative humidity) and 160 ppm or greater, respectively, for a prescribed time. Specifically, the chamber interior is kept at positive pressure (10-20 Pa) by the treatment illustrated in the flow chart of Fig. 3. The control based on this flow chart is repeatedly accomplished by the control unit 58 at micro time intervals. Positive pressure means a positive value for (chamber interior pressure) - (exterior chamber pressure).

First, the control unit 58 obtains the pressure difference between the inside and outside of the chamber as detected by the fine pressure difference detector 56 (step S10), and stores it in the memory device 84 (step S11). A pressure difference of 10-20 Pa (step S12) is ordinary pressure, and therefore the flow returns to the processing of step S10, and for continued processing of pressure difference detection (step S10) and detected value storage (Step S11).

However, a pressure difference of less than 10 Pa (step S12) between the inside and outside of the chamber as detected by the fine pressure difference detector 56 indicates that the chamber pressure is too low, and therefore air is supplied into the chamber (step S14). That is, a control signal is sent to the supply air volume regulating electromagnetic valves 62 and air fan 66 to open the supply air volume regulating electromagnetic valves 62 for a prescribed time and to activate the air fan 66. This supplies air from outside the chamber into the chamber through the supply air volume regulating electromagnetic valves 62 and HEPA filter 64, and causes the pressure in the chamber to increase by a value corresponding to the time the supply air volume regulating electromagnetic valves 62 are opened. If the pressure difference between the inside and outside of the chamber as detected by the fine pressure difference detector 56 is still less than 10 Pa upon completion of air supply into the chamber, the air supply procedure is carried out again (steps S10-S12, step S14).

A pressure difference between the inside and outside of the chamber as detected by the fine pressure difference detector 56 of greater than 20 Pa (step S12) indicates that the pressure in the chamber is too high, and therefore air is discharged to the outside (step S13). That is, a control signal is sent to the discharge gas volume regulating electromagnetic valves 74 and air fan 82 to open the discharge gas volume regulating electromagnetic valves 74 for a prescribed time and to activate the air fan 82. This discharges gas in the chamber to the outside through the HEPA filter 72, discharge gas volume regulating electromagnetic valves 74 and air treater 76, and causes the pressure in the chamber to decrease by a value corresponding to the time the discharge gas volume regulating electromagnetic valves 74 are opened.

If the pressure difference between the inside and outside of the chamber as detected by the fine pressure difference detector 56 is still 20 Pa or greater upon completion of discharge gas discharge to the outside, the discharge gas discharge procedure is carried out again (steps S10-S13).

Since this chamber pressure controller 6 allows the pressure difference between the inside and outside of the chamber to be kept at a constant 10-20 Pa, when formaldehyde gas is used for disinfection of the chamber it is possible to prevent leakage of untreated formaldehyde gas to the outside even when the volume of the gas in the chamber increases due to increasing chamber temperature, since the formaldehyde gas is discharged after being treated by the air treater 76. Also, since the detected pressure difference between the inside and outside of the chamber is stored in the memory device 84 in a time-serial manner, the detected value stored in the memory device 84 may be outputted by the output device 86 to ensure that the pressure in the chamber is constantly kept at the prescribed positive pressure, based on the output results. It is thus possible to ensure that untreated formaldehyde gas is not leaking out of the chamber.

After the temperature, humidity and formaldehyde gas concentration in the chamber have been kept in the temperature range of 20-40°C, humidity range of 50-90% (relative humidity) and formaldehyde gas concentration range of 160 ppm or greater for the prescribed time, the formaldehyde gas generator 36 is switched off and treatment is carried out with the discharge gas treater 46 until the formaldehyde concentration in the chamber falls to below a prescribed value.

In the embodiment described above, the chamber pressure regulator 6 is provided with an air treater 76, but treatment of the formaldehyde gas may also be accomplished using the discharge gas treater 46 of the formaldehyde gas supply/ejection apparatus 4.

The apparatus of the invention has an integral housing structure and is mounted outside the disinfection space, while formaldehyde gas is introduced into the disinfection space and discharge gas from the disinfection space is treated to produce clean discharge gas. The apparatus is also demountable. The apparatus of the invention can also be easily moved to the location of the disinfection space, thus allowing adequate disinfection which can easily ensure the sterility of ambulance rooms, mobile operating rooms (including tents) and bioclean rooms.

The apparatus of the invention also leaves no residue of formaldehyde gas in the disinfection space after disinfection treatment and substances in the disinfection space are not corroded, such that disinfection of precision instruments can also be accomplished.

Moreover, since the apparatus of the invention allows the formaldehyde gas to be supplied from the formaldehyde gas inlet while exhibiting a high disinfecting effect, it is possible to easily guarantee adequate disinfection of the interior spaces of artificial respirators.

In addition, the apparatus of the invention can prevent leakage of untreated formaldehyde gas outside of the chamber even when the volume of the gas in the chamber increases due to rising chamber temperature, while also adequately guaranteeing the disinfecting effect.

### INDUSTRIAL APPLICABILITY

As explained above, the formaldehyde gas disinfection apparatus of the invention is suitable for disinfection to a degree which can adequately guarantee disinfection spaces.

## Claims

1. A formaldehyde gas disinfection apparatus comprising
(A) a formaldehyde gas supply/ejection apparatus which supplies and ejects formaldehyde gas into and from a sealed chamber (100), said formaldehyde gas supply/eject apparatus comprising:
(i) a formaldehyde gas generator (36) which generates the formaldehyde gas,
(ii) a humidity regulator (32) which regulates the humidity of the formaldehyde gas,
(iii) a temperature regulator (34) which regulates the temperature of the formaldehyde gas,
(iv) a gas deliverer (26) which delivers and introduces the formaldehyde gas into the chamber,
(v) a discharge gas treater (46) which treats discharge gas from the chamber,
(vi) a gas ejector (28) which ejects the discharge gas, and
(vii) a controller (24) which controls the formaldehyde gas concentration, humidity and temperature in the chamber to a prescribed concentration, humidity and temperature,
and
(B) a chamber pressure regulator (6) which regulates the pressure in the chamber, said chamber pressure regulator comprising:
(viii) an air supply unit (52) which supplies outside air into the chamber,
(ix) a gas discharge unit (54) which discharges gas in the chamber to the outside,
(x) pressure difference detector means (56) which detects the pressure difference between the inside and outside of the chamber, and
(xi) control means (58) which controls the air supply unit and the gas discharge unit based on a detected value detected by the pressure difference detector means.

2. The apparatus of claim 1, wherein the chamber pressure regulator (B) additionally comprises:
(xii) controlled state output means (86) which outputs the controlled state of the chamber pressure based on the detected value detected by the pressure difference detector means.

3. The apparatus of claim 1 or 2, wherein the controller (vii) controls the formaldehyde gas concentration, humidity and temperature in the chamber to 160 ppm or more, 50-90% RH and 20-40°C, respectively, based on the values of formaldehyde gas concentration, humidity and temperature obtained from monitors in the chamber.

4. The apparatus of any one of claims 1 to 3, wherein the gas discharge unit (ix) is provided with a treatment apparatus (76) which treats gas discharged from the chamber.

## Patentansprüche

1. Desinfektionsvorrichtung mit Formaldehydgas, die Folgendes umfasst:
(A) eine Formaldehydgas-Zufuhr/Ausstoßvorrichtung, die Formaldehydgas einer versiegelten Kammer (100) zuführt und daraus ausstößt, wobei die Formaldehydgas-Zufuhr/Ausstoßvorrichtung Folgendes umfasst:
(i) einen Formaldehydgas-Erzeuger (36), der das Formaldehydgas erzeugt,
(ii) einen Feuchtigkeitsregulator (32), der die Feuchtigkeit des Formaldehydgases reguliert,
(iii) einen Temperaturregulator (34), der die Temperatur des Formaldehydgases reguliert,
(iv) einen Gasüberträger (26), der das Formaldehydgas in die Kammer überträgt und einführt,
(v) einen Abgasbehandler (46), der Abgas aus der Kammer behandelt,
(vi) einen Gasausstoßer (28), der das Abgas ausstößt, und
(vii) einen Regler (24), der die FormaldehydgasKonzentration, Feuchtigkeit und Temperatur in der Kammer auf eine vorgeschriebene Konzentration, Feuchtigkeit und Temperatur regelt, und
(B) einen Kammerdruckregulator (6), der den Druck in der Kammer reguliert, wobei der Kammerdruckregulator Folgendes umfasst:
(viii) eine Luftzufuhreinheit (52), die der Kammer Außenluft zuführt,
(ix) eine Gasablasseinheit (54), die Gas in der Kammer nach außen ablässt,
(x) ein Druckdifferenzdetektormittel (46), das die Druckdifferenz zwischen dem Inneren und Äußeren der Kammer detektiert, und
(xi) ein Regelmittel (58), das die Luftzufuhreinheit und die Gasablasseinheit auf Basis eines detektierten Wertes, der durch das Druckdifferenzdetektormittel detektiert wird, regelt.

2. Vorrichtung gemäß Anspruch 1, worin der Kammerdruckregulator (B) zusätzlich Folgendes umfasst:
(xii) ein Ausgabemittel des geregelten Zustands (86), das den gesteuerten Zustand des Kammerdrucks auf Basis des detektierten Wertes, der vom Druckdifferenzdetektormittel detektiert wird, ausgibt.

3. Vorrichtung gemäß Anspruch 1 oder 2, worin der Regler (vii) die Formaldehydgaskonzentration, Feuchtigkeit und Temperatur in der Kammer auf 160 ppm oder mehr, 50-90 % r.F. bzw. 20-40°C auf Basis der Werte der Formaldehydgaskonzentration, Feuchtigkeit und Temperatur regelt, die von Überwachungseinrichtungen in der Kammer erhalten werden.

4. Vorrichtung gemäß einem der Ansprüche 1 bis 3, worin die Gasablasseinheit (ix) mit einer Behandlungsvorrichtung (76) versehen ist, die aus der Kammer abgelassenes Gas behandelt.

## Revendications

1. Appareil de désinfection au gaz formaldéhyde comprenant :
(A) un appareil d'alimentation/éjection de formaldéhyde qui alimente et éjecte du gaz formaldéhyde dans et à partir d'une chambre étanche (100), ledit appareil d'alimentation/éjection de gaz formaldéhyde comprenant :
(i) un générateur de gaz formaldéhyde (36) qui génère le gaz formaldéhyde,
(ii) un régulateur d'humidité (32) qui régule l'humidité du gaz formaldéhyde,
(iii) un régulateur de température (34) qui régule la température du gaz formaldéhyde,
(iv) un fournisseur de gaz (26) qui fournit et introduit le gaz formaldéhyde dans la chambre,
(v) un élément de traitement de gaz d'évacuation (46) qui traite le gaz d'évacuation provenant de la chambre,
(vi) un éjecteur de gaz (28) qui éjecte le gaz d'évacuation, et
(vii) un élément de contrôle (24) qui contrôle la concentration de gaz formaldéhyde, l'humidité et la température dans la chambre à une concentration, une humidité et une température prescrites,
et
(B) un régulateur de pression de chambre (6) qui régule la pression dans la chambre, ledit régulateur de pression de chambre comprenant :
(viii) une unité d'alimentation en air (52) qui alimente la chambre en air extérieur,
(ix) une unité d'évacuation de gaz (54) qui évacue le gaz de la chambre vers l'extérieur,
(x) un moyen de détection de la différence de pression (56) qui détecte la différence de pression entre l'intérieur et l'extérieur de la chambre, et
(xi) un moyen de contrôle (58) qui contrôle l'unité d'alimentation en air et l'unité d'évacuation de gaz basé sur une valeur détectée qui est détectée par le moyen de détection de différence de pression.

2. Appareil selon la revendication 1, dans lequel le régulateur de pression de chambre (B) comprend additionnellement:
(xii) un moyen de sortie à l'état contrôlé (86) qui produit l'état contrôlé de la pression de chambre basée sur la valeur détectée qui est détectée par le moyen détecteur de différence de pression.

3. Appareil selon la revendication 1 ou 2, dans lequel l'élément de contrôle (vii) contrôle la concentration de gaz formaldéhyde, l'humidité et la température dans la chambre à 160 ppm ou plus, 50 à 90 % d'humidité relative et 20 à 40 °C, respectivement, sur base des valeurs de la concentration de gaz formaldéhyde, de l'humidité et de la température obtenues à partir de moniteurs dans la chambre.

4. Appareil selon l'une quelconque des revendications 1 à 3, dans lequel l'unité d'évacuation de gaz (ix) est pourvue d'un appareil de traitement (76) qui traite le gaz évacué de la chambre.
